# EUROPEAN PATENT SPECIFICATION

(11) **EP 0 359 789 B1**
(45) Date of publication and mention of the grant of the patent: **04.08.1993**
(21) Application number: 89902420.2
(22) Date of filing: 12.01.1989
(51) Int. Cl.: C12Q 1/68

(54) **AMPLIFICATION AND DETECTION OF NUCLEIC ACID SEQUENCES**
VERSTÄRKUNG UND NACHWEIS VON NUKLEINSÄURESEQUENZEN
AMPLIFICATION ET DETECTION DE SEQUENCES D'ACIDES NUCLEIQUES

(30) Priority: 21.01.1988 US 146462
(43) Date of publication of application: 28.03.1990
(73) Proprietor: GENENTECH, INC., South San Francisco California 94080 (US)
(72) Inventor: MILLER, Harvey, I., Pleasant Hill, CA 94523 (US); JOHNSTON, Sean, Los Angeles, California 90025 (US)
(74) Representative: Armitage, Ian Michael
(86) International application number: US8900120
(87) International publication number: WO8906700

(56) References cited:
- EP-A- 310 229
- WO, A1, 88/10315 (Siska Diagnostics, Inc.) 29 December 1988
- Science Vol. 239, 1988 E.S. Stoflet et al: "Genomic Amplification with Transcript Sequencing", page 491-492
- Nucleic Acids Research, Vol. 15, No 21, 1987 John F. Milligan et al: "Oligoribonucleotide synthesis using T7 RNA polymerase and synthetic DNA templates", page 878 and page 8798

## Description

### Background of the Invention

Recent developments in the general field of molecular biology have made possible the detection of specific nucleic acid sequences of clinical and commercial importance. Analysis of the nucleic acid sequences of various human genes, for example, has revealed unique sequence alterations that are associated with specific diseases. Likewise, sequences have been identified within the genome of various pathogens which uniquely characterize each organism, and distinguish them from even closely related species. The availability of such sequence information has made possible the diagnosis of diseases at the genetic level.

The most common method for the detection of a specific nucleic acid sequence is hybridization. This method takes advantage of the ability of a nucleic acid sequence to form a stable noncovalent complex with a complementary nucleic acid sequence. In order to determine whether a specific nucleic acid sequence is present within a test sample, a complementary nucleic acid probe is prepared, labeled with a detectable chemical modification, and then added to the test sample. If the sequence to be detected is present, the labeled probe will become hybridized to it, with the detectable label providing the means for determining in a known manner whether hybridization has occurred, and to what extent.

The principal limitation on the use of present hybridization methods for the determination of a nucleic acid in a test sample is that they are not sensitive enough and therefore require a relatively large amount of sample to accurately verify the presence of a specific nucleic acid sequence. This limitation presents significant practical difficulties in a clinical setting because of the limited quantity of sample that is typically available for analysis. Consequently, much attention has been focused on developing methods for improving the sensitivity of the hybridization method for detecting a specific nucleic acid sequence in a test sample and thereby expanding its utility in the diagnostic arts.

One general approach to improving the sensitivity of the hybridization method has been to increase the signal generated by the hybridization probe. Methods have been described, for example, for preparing nucleic acid probes labeled to high specific activity with radioactive labels, either by nick-translation (Rigby, et al., 1977, J. Mol. Biol. 113:237) or by SP6 transcription (Melton, et al., 1984, Nuc. Acids Res. 12:7035).

Schneider, et al., PCT Pat. App. WO 87/03622, describe the use of multiple signal-generating secondary probes, each capable of binding to a primary probe which has hybridized to a DNA or RNA sequence of interest, as a means for amplifying the signal generated by the hybridization event.

Chu, et al., PCT Pat. App. WO 87/06270 describe the use of "replicative RNA", alone or in conjunction with an affinity molecule, as a nucleic acid hybridization probe, and the use of an RNA-dependent RNA polymerase to replicate, and thereby increase the signal generated by, the bound probe RNA.

Rodland, et al., U.S. Pat. No. 4,647,529, teach the use of thionucleotides, which when incorporated into a hybridization probe increase the amount of binding between the probe and the nucleic acid sequence to be detected.

In contrast to the substantial efforts that have been made to increase the sensitivity of probe detection methods, relatively little research has been devoted to methods of amplifying the nucleic acid sequence to be detected in a test sample so that it is produced in sufficient quantities to be detected by currently available hybridization methods.

Mullis, et al., U.S. Pat. No. 4,683,195, teach a process for amplifying a specific nucleic acid sequence in a test sample by the use of two oligonucleotide primers, and the synthesis of primer extension products. The extension product of one primer, when hybridized to the other primer, becomes a template for production of the desired specific nucleic acid sequence, and vice versa, and this process is repeated as often as necessary to produce the desired amount of the specific sequence.

Mullis, et al. also teach the use of this amplification method to prepare large quantities of a recombinant nucleic acid, using primers to which a non-complementary sequence is ligated. Mullis, et al. disclose ligating a nucleotide sequence for a promoter, linker, or coding sequence to one or both primers, whereby the non-complementary sequence is amplified together with the test sample nucleic acid sequence. In this manner, a large quantity of a recombinant nucleic acid is produced, consisting of an existing test sample nucleic acid sequence ligated to a selected exogenous nucleic acid sequence.

The essence of the Mullis, et al. process, therefore, is the amplification of a desired nucleic acid sequence in the form of complementary primer extension products. Because each primer extension product formed serves as the template for synthesis of yet another primer extension product, multiple repetitions of the Mullis, et al. primer extension procedure theoretically should result in the accumulation of the desired sequence at an exponential rate relative to the number of reaction cycles. In contrast, by-products formed by primer hybridizations other than those intended are not expected to be self-catalytic, and should therefore accumulate at a linear rate.

The specificity and accuracy of the Mullis, et al. process is thus seen to be strictly dependent upon the substantially increased rate of synthesis of the desired nucleic acid sequence relative to that of by-products. In practice, however, it is observed that the accumulation of by-products may proceed at a much greater rate than that predicted by theoretical calculations. Mullis et al., for example, disclose the use of their process to amplify a portion of the human beta-hemoglobin gene which is present in a sample containing human genomic DNA, and find that due to amplification of other sequences within the genome, only 1% of the final resulting population of amplified sequences correspond to the desired sequence.

To the extent that the Mullis, et al. process results in the amplification of nucleic acid sequences other than the one desired, the method assays a greater amount of the desired nucleic acid sequence than is actually present in the test sample, and produces false-positive results. It is one object of the present invention, therefore, to provide a method for specifically and quantitatively amplifying a desired nucleic acid sequence in a test sample, thereby improving the sensitivity of detection of the desired sequence, while avoiding the problems of false-positive and otherwise inaccurate results inherent in the existing technology.

Furthermore, the Mullis, et al. process contemplates multiple cycles of primer hybridization and primer extension synthesis, resulting in burdensome labor or automation costs. Automation of the Mullis, et al. process not only necessitates the use of specialized equipment but also the use of a specialized reagent, a heat-stable DNA polymerase, and reaction conditions tailored to each analyte test system so that the primer hybridization and primer extension synthesis steps may be conducted continuously. It is thus a further object of the present invention to provide a method for detecting a specific nucleic acid sequence in a test sample which is rapid and simple to perform without costly, repetitious reaction steps, specialized equipment and reagents, or burdensome requirements for changing reaction conditions for each different test sample.

### Summary of the invention

According to the present invention there is provided a method for preparing a double-stranded nucleic acid which includes a promoter operably linked to a sequence to be detected, which method comprises:
(a) providing an oligonucleotide promoter-primer;
(b) contacting said promoter-primer with a nucleic acid comprising the sequence to be detected under conditions that permit hybridization of the promoter-primer to the nucleic acid sequence to be detected;
(c) synthesizing an extension product from the 3' end of the promoter-primer, which extension product is complementary to the nucleic acid sequence to be detected;
(d) contacting the product of step (c) with an agent possessing 3'-5' exonuclease activity; and
(e) synthesizing an extension product from the 3' end of the sequence to be detected, which extension product is complementary to the promoter of the promoter-primer, wherein the promoter-primer of step (a) is the only primer utilized.

The present invention is directed to improved methods for determining the presence of a specific nucleic acid sequence in a test sample, and kits containing reagents necessary for practicing the invention. In general, the methods of the invention involve the synthesis of a double-stranded nucleic acid containing the nucleic acid sequence to be detected and a promoter, the synthesis of a multiplicity of RNA transcripts under the control of the promoter, and the detection of the specific RNA transcripts produced. The advantage obtained by amplification in the form of RNA transcripts rather than DNA primer extension products is that the synthesis of RNA transcripts, in the presence of an enzyme for polymerization and ribonucleoside triphosphates, occurs continuously, thus producing any desired level of amplification of the nucleic acid sequence to be detected without resort to repeated cycles of exacting and inherently error-prone primer hybridization reactions.

### Brief Description of the Drawing

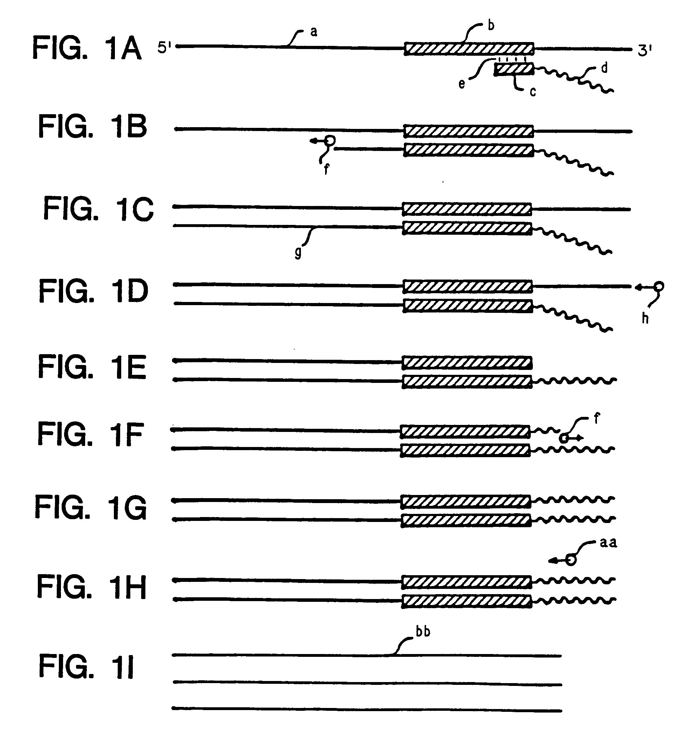

Fig. 1 illustrates the hybridization of an oligonucleotide promoter-primer to a specific sequence to be detected within a single-stranded nucleic acid, synthesis of a double-stranded nucleic acid which comprises the sequence to be detected and the promoter of the promoter-primer, and the synthesis of a multiplicity of RNA transcripts from the double-stranded nucleic acid, under the control of the promoter.

### Detailed Description of the Invention

The term "promoter" as used herein refers to a nucleic acid sequence at which an RNA polymerase enzyme binds and initiates the synthesis of RNA transcripts. The promoter is preferably a dedicated promoter, and the RNA polymerase is preferably a dedicated RNA polymerase. The term "dedicated" refers to the ability of the promoter to be recognized substantially only by an RNA polymerase other than that which is typically present in the test sample to be assayed. Similarly, the dedicated RNA polymerase will bind only to or preferentially to the dedicated promoter as compared to other promoters present in the test sample, and synthesize RNA transcripts of any nucleic acid sequence located downstream from the promoter. The combination of a dedicated promoter and a dedicated RNA polymerase results in an unusually specific promoter-RNA polymerase interaction.

Typically, the dedicated promoter and dedicated RNA polymerase will be obtained from the same source, as for example bacteriophage T7 or bacteriophage SP6. In the case of both T7 and SP6, the phage encoded RNA polymerase efficiently initiates synthesis of RNA transcripts only at the cognate phage promoter. RNA transcripts resulting from initiation at other procaryotic or eucaryotic promoters are rarely observed. Moreover, the transcription reaction, consisting of a simple salt buffer, double-stranded nucleic acid template, ribonucleoside triphosphates, and phage RNA polymerase, results in rapid synthesis of large amounts of RNA. The promoter is desirably selected or its nucleotide sequence modified from the native sequence in order to minimize any adventitious hybridization to any known sequences in the test sample DNA.

The term "primer" as used herein refers to an oligonucleotide sequence, whether occurring naturally or produced synthetically, which is substantially complementary or homologous to all or part of a nucleic acid sequence to be detected. The primer must be sufficiently long to hybridize with a template nucleic acid containing the sequence to be detected or its complement, and to prime the synthesis of an extension product in the presence of an agent for polymerization. The exact length of the primer will depend on many factors, including hybridization and primer extension synthesis reaction conditions, and the composition of the specific nucleic acid sequence to be detected. Typically the primer will contain 10-25 or more nucleotides, although it may contain fewer nucleotides. It is not necessary, however, that the primer reflect the exact sequence of the nucleic acid sequence to be detected or its complement. For example, non-complementary bases can be interspersed into the primer, or complementary bases deleted from the primer, provided that the primer is capable of hybridizing specifically with the nucleic acid sequence to be detected, or its complement, under the conditions chosen.

The term "promoter-primer" as used herein refers to an oligonucleotide whether occurring naturally or produced synthetically which comprises a promoter joined to the 5' end of a primer. Under suitable conditions and in the presence of an agent for polymerization, the promoter-primer is capable of acting as a point of initiation of a promoter-primer extension product that includes the nucleic acid sequence to be detected, or its complementary sequence, and the promoter of the promoter-primer. The promoter-primer is preferably single-stranded for maximum efficiency of hybridization, but may alternatively be double-stranded. If double-stranded, the promoter-primer is first treated to separate its strands before being used to prepare extension products.

The term "probe" as used herein refers to an oligonucleotide whether occurring naturally or produced synthetically, which is either homologous or complementary to all or part of a nucleic acid sequence to be detected. The probe is preferably selected so that under appropriate conditions it is capable of hybridizing specifically to RNA transcripts of the nucleic acid sequence to be detected.

The term "oligonucleotide" as used herein in reference to primers and probes is defined as a nucleic acid molecule comprised of two or more deoxyribonucleotides or ribonucleotides. A desired oligonucleotide may be prepared by any suitable method, such as purification from a naturally occurring nucleic acid, or de novo synthesis. Several methods have been described in the literature, for example, for the synthesis of oligonucleotides from nucleoside derivatives using various techniques of organic chemistry. One type of organic synthesis is the phosphotriester method, wherein phosphotriester nucleosides are joined together to form an oligonucleotide with a desired sequence (Narang, et al.. 1979, Meth. Enzymol. 68:90). Other methods of organic synthesis which have been described involve the use of phosphodiester nucleosides (Brown, et al., 1979, Meth. Enzymol. 68:109), or phosphoramidate nucleosides (Caruthers, et al., 1985, Meth. Enzymol. 154:287). Oligonucleotides synthesized by any of these methods may subsequently be joined together to form a single oligonucleotide of any required length and sequence. Alternatively, oligonucleotides are produced by in vitro transcriptional amplification, by cloning in host cells or by recovery from natural sources by the use of appropriate restriction enzymes.

The term "RNA transcript" as used herein refers to a ribonucleic acid molecule synthesized by an RNA polymerase enzyme under the control of the promoter of the promoter-primer. The RNA transcript of a specific nucleic acid sequence to be detected is either homologous or complementary to that sequence, depending upon the nature of the promoter-primer.

The term "extension product" as used herein refers to a nucleic acid molecule, the synthesis of which is initiated at the 3'-OH terminus of a primer, using as a template for synthesis the nucleic acid molecule to which the primer is hybridized.

The term "agent for polymerization" as used herein is generally understood to refer to any enzyme that catalyzes the synthesis of a nucleic acid molecule from deoxyribonucleotides or ribonucleotides, using an existing nucleic acid as a template.

Any source of nucleic acid, in purified or non-purified form, can be utilized as the test sample. For example, the test sample may be a food or agricultural product, or a human or veterinary clinical specimen. Typically, the test sample is a biological fluid such as urine, blood, plasma, serum, sputum or the like. The nucleic acid to be detected in the test sample is DNA or RNA, including messenger RNA, from any source, including bacteria, yeast, viruses, and the cells or tissues of higher organisms such as plants or animals. Methods for the extraction and/or purification of such nucleic acids have been described, for example, by Maniatis, et al., Molecular Cloning: A Laboratory Manual (New York, Cold Spring Harbor Laboratory, 1982).

The nucleic acid sequence to be detected in the test sample may be present initially as a discrete molecule so that the sequence to be detected constitutes the entire nucleic acid, or may only be a component of a larger molecule. It is not necessary that the nucleic acid sequence to be detected be present initially in a pure form. The test sample may contain a complex mixture of nucleic acids, of which the nucleic acid sequence to be detected comprises a minor fraction. For example, the nucleic acid sequence to be detected may correspond to an oncogene contained in total human genomic DNA, or a portion of the nucleic acid sequence of a pathogenic organism which organism is a minor component of a clinical sample.

Any nucleic acid sequence may be detected by the present invention. It is only necessary that a sufficient number of nucleotides of the sequence be known so that at least one and preferably two primers may be prepared that are capable of hybridizing with the nucleic acid sequence to be detected or its complement. The nucleic acid sequence to be detected may be ascertained by any of the known methods of nucleic acid sequencing, for example, or may be predicted based upon a determined protein sequence. The greater the knowledge of the nucleic acid sequence to be detected, the greater can be the specificity of the primers selected, and thus the greater the accuracy of the invention.

Sufficient sequence information should be available, for example, to allow the selection of a promoter-primer which will hybridize specifically with the nucleic acid sequence to be detected, or its complement, but no other sequence in the test sample, under the hybridization conditions chosen.

Referring to the method depicted in Fig. 1, the core initial step is the preparation of a double-stranded nucleic acid which includes the sequence to be detected and a promoter. In general, the preparation of this double-stranded nucleic acid involves the synthesis of a promoter-primer extension product using as a template a single-stranded nucleic acid to which the primer has hybridized. The template will therefore comprise a test sample nucleic acid which contains the sequence to be detected.

If the template is originally contained in a double-stranded nucleic acid, it is necessary to separate the strands of the nucleic acid before or simultaneous with the synthesis of a primer extension product. Strand separation is desirably accomplished by heat denaturation, wherein the test sample is heated to about 90°-100°C for times ranging from about 1-10 minutes, although other physical, chemical, or enzymatic denaturation procedures may be used.

Primer hybridization is typically performed in a buffered aqueous solution, for which the conditions of temperature, salts concentration, and pH are selected to provide sufficient stringency such that the primer will hybridize specifically to the nucleic acid sequence to be detected, or its complement, but not any other sequence. Generally, the efficiency of hybridization between primer and template will be improved under conditions where the amount of primer added is in molar excess to the template, preferably a 1000 to 10⁶ molar excess. It is understood, however, that the amount of template in the test sample may not be known, so that the amount of primer relative to template cannot be determined with certainty.

Fig. 1A-G depict one embodiment of the present invention wherein a promoter-primer is used to prepare a double-stranded nucleic acid which includes the sequence to be detected and a promoter.

In Fig. 1A, the primer c of the promoter-primer c d is shown hybridizing with the nucleic acid sequence to be detected b as part of a nucleic acid a, through non-covalent base pairing e of complementary nucleotide sequences. The promoter d of the promoter-primer, being preferably selected so as not to be complementary with any known nucleic acid sequence in the test sample, remains unhybridized.

The subsequent addition of a polynucleotide polymerase f, in the presence of nucleoside triphosphates, results (Fig. 1B-C) in the synthesis of a promoter-primer extension product g, initiating at the 3' end of the primer and proceeding in the 5' direction along the single-stranded template, which template consists of the test sample nucleic acid sequence a flanking on the 5' side of the sequence b within which the primer is originally hybridized. The primer extension product is thus complementary to the nucleic acid sequence to be detected and the 5'-flanking test sample nucleic acid sequence, if any, forming a double-stranded molecule.

In the presence of an agent possessing 3'-5' exonuclease activity h, the single-stranded sequence at the 3' end of the test sample nucleic acid, if any, is excised as shown in Fig. 1D, and replaced by new nucleic acid (Fig. 1E-G), the synthesis of which is initiated by a polynucleotide polymerase f at the 3' end of the test sample nucleic acid using as a template the promoter of the promoter-primer.

The method depicted in Fig. 1A-G for generating a double-stranded molecule which includes the sequence to be detected and a promoter depends upon hybridization of a promoter-primer to the nucleic acid sequence to be detected and subsequent steps of promoter-primer extension synthesis, nucleic acid excision, and replacement synthesis. Although the excision reaction may be carried out by an agent h different from that used for promoter-primer extension synthesis and replacement synthesis f, it is desirable to use a single agent such that the synthesis of the double-stranded product may be carried out in a continuous manner following promoter-primer hybridization. Accordingly, the polynucleotide polymerase is preferably one which possesses both 5'-3' polymerase activity and 3'-5' exonuclease activity, such as, for example, E. coli DNA polymerase I, Klenow fragment of E. coli DNA polymerase I, and T4 DNA polymerase. As each of these enzymes requires a DNA template for primer extension synthesis, it is understood that their use according to the method depicted in Fig. 1B and 1F will be appropriate in instances where the nucleic acid sequence to be detected is DNA.

The double-stranded nucleic acid which includes the sequence to be detected and a promoter serves as the source of a multiplicity of RNA transcripts of the sequence to be detected. As depicted in Fig. 1H-I, addition of a suitable RNA polymerase aa to the test sample results in the binding of the RNA polymerase to the promoter of the double-stranded product, and in the presence of ribonucleoside triphosphates, the subsequent synthesis of RNA transcripts bb of the nucleic acid sequence located downstream from the promoter, including the nucleic acid sequence to be detected.

Under suitable reaction conditions, including the presence of the necessary reagents,. the synthesis of RNA transcripts will occur continuously and in proportion to the amount of the nucleic acid sequence to be detected that was originally present in the test sample. Additional reagents may be added as necessary to prepare the desired quantity of RNA transcripts. Preferably the synthesis of RNA transcripts will be carried out in the presence of a ribonuclease inhibitor, as for example vanadyl-ribonucleoside complexes or human placental ribonuclease inhibitor, in order to avoid possible degradation of the transcripts by any adventitious ribonuclease contaminant. Berger, 1987, Meth. Enzymol. 152:227; de Martynoff et al., 1980, Biochem. Biophys. Res. Commun. 93:645; Sheel et al., 1979, Proc. Natl. Acad. Sci. 76:4898. After the appropriate length of time has passed to produce the desired quantity of RNA transcripts, the reaction may be halted by inactivating the RNA polymerase in any known manner or separating the components of the reaction.

Determination of the specific RNA transcripts produced is accomplished by any suitable method, as for example, by labeling of the RNA transcripts with a detectable moiety during or after their synthesis, or by the use of a labeled probe capable of hybridizing specifically with RNA transcripts of the nucleic acid sequence to be detected.

RNA transcripts may be labeled, for example, by providing ribonucleoside triphosphates which are themselves labeled with a detectable moiety, and which are utilized as a substrate by RNA polymerase and thereby incorporated into RNA transcripts. The detectable moiety can be any one which is capable of producing, either directly or indirectly, a detectable signal. In one embodiment, for example, the detectable moiety may be a radioisotope, such as ³²P, ³H, ¹⁴C, ¹²⁵I, and ³⁵S. In a further embodiment, the detectable moiety may be a non-radioactive chemical modification capable of producing a detectable signal upon interaction with one or more appropriate agents. Thus, for example, the detectable moiety can be biotin, and the detectable signal produced by the formation of a complex between the biotin moiety and a protein capable of binding specifically to biotin, as for example avidin, streptavidin, or anti-biotin antibody, which binding protein is conjugated with a detectable molecule, like fluorescein, or with an enzyme which reacts with a suitable substrate to form a fluorescent, luminescent, or colored product. Langer et al., 1981, Proc. Natl. Acad. Sci. 78:6633; Bayer and Wilchek, 1980, Meth. Biochem. Anal. 26:1.

In an additional embodiment, the specific RNA transcript produced may be determined by the use of a nucleic acid hybridization probe. Falkow et al., U.S. Pat. No. 4,358,535; Goodson et al., European Pat. App. 0 238 332. The probe is desirably selected so as to hybridize within the sequence of the RNA transcript which is either complementary or homologous to the nucleic acid sequence to be detected. The hybridization method may be carried out by any suitable method, including the liquid hybridization methods described in European Pat. Publication Nos. 70,685 and 70,687, and the liquid-solid hybridization methods described in U.S. Pat. Nos. 4,358,535 (Falkow) and 4,647,529 (Rodland), European Pat. App. 0 238 332 (Goodson), and Ranki, et al., 1983, Gene 21:77. In the case of liquid-solid hybridization, either the probe molecules or the RNA transcripts can be immobilized on the solid support.

Any suitable method may be used for correlating the amount of RNA transcripts with the amount of nucleic acid sequence to be detected in the test sample, including, for example, determination of a fixed quantity of a standard nucleic acid which standard nucleic acid contains the sequence to be detected or another known sequence, simultaneous or in parallel with determination of the sequence to be detected in the test sample. Ranki, et al., UK Pat. App. 2 187 283 A.

The following example is offered by way of illustration, and is not intended to limit the invention in any manner.

### Example 1

This example illustrates the use of a deoxyribonucleotide promoter-primer having the sequence
5'AAATTAATACGACTCACTATAGGGAGATGTACCTCTGTATCATATGC 3'
for the detection of the env gene of HIV (human immunodeficiency virus; formerly called HTLV-III/LAV). The sequence of this promoter-primer is selected such that the 5' end of the promoter-primer corresponds to the sequence of the functional domain of a bacteriophage T7 class III promoter (Dunn and Studier, 1983, J. Mol. Biol. 166:477), and the 3' end is complementary to a portion of the coding sequence of the env gene of HIV, between nucleotides 5981-6000 of the HIV genomic sequence (Muesing et al., 1985, Nature 313:450).

Nucleic acid for analysis is extracted from test samples of citrate-treated human blood or virus-infected H9 cells (Popovic, et al., 1984, Science 224:497) using the techniques described by Hermann and Fischauf, 1987, Meth. Enzymol. 152:180.

A total of 10 µg of test sample nucleic acid is added to 100 pmoles of promoter-primer in 100 µl of reaction buffer containing 40mM Tris HCl (pH 8.0), 20mM MgCl₂, 1mM dithiothreitol, 5 mg/ml gelatin, 10mM vanadyl-ribonucleoside complexes, and 10mM each of the four deoxyribonucleoside triphosphates (dATP, dTTP, dGTP, dCTP) and the four ribonucleoside triphosphates (ATP, UTP, GTP, CTP). The mixture is heated at 100°C for one minute, and allowed to cool to 37°C. 5 units of Klenow fragment of E. coli DNA polymerase I and 5 units of T7 RNA polymerase are then added to the mixture and the reactions allowed to proceed for one hour at 37°C.

The RNA transcripts produced are detected by dot-blot hybridization, using as a hybridization probe a ³²P end-labeled deoxyoligonucleotide having the sequence
5' TTGATGATCTGTAGTGCTAC 3'.
The sequence of this probe was selected to be homologous to a portion of the coding sequence of the HIV env gene, located between nucleotides 5875 and 5895 of the HIV genomic sequence.

Aliquots from the RNA synthesis reaction are added to 100 µl of 15X SSC buffer (1X SSC = 0.15M sodium chloride, 0.015M sodium citrate, pH 7.0) and filtered through nitrocellulose filters prewet in 6X SSC. The filters are then baked for one hour at 80°C in a vacuum oven.

After baking, each filter is contacted with hybridization solution consisting of 6X SSC, 50mM sodium phosphate (pH 6.5), 5X Denhardt's solution (1X = 0.02% polyvinylpyrrolidone, 0.02% Ficoll, 0.02% bovine serum albumin, 0.2mM Tris HCl, 0.2mM EDTA, pH 8.0), 0.1% sodium dodecyl sulfate, and 0.2% denatured salmon sperm DNA for one hour at 42°C. The labeled probe is then added to the hybridization solution to a final concentration of 10⁷ cpm/ml and incubation of the filters continued for two hours at 42°C.

Finally, the filters are washed in 6X SSC for one hour at 37°C with two changes of buffer. After drying, the filters are autoradiographed or counted for Cerenkov radioactivity.

## Claims

1. A method for preparing a double-stranded nucleic acid which includes a promoter operably linked to a sequence to be detected, which method comprises:
(a) providing an oligonucleotide promoter-primer;
(b) contacting said promoter-primer with a nucleic acid comprising the sequence to be detected under conditions that permit hybridization of the promoter-primer to the nucleic acid sequence to be detected;
(c) synthesizing an extension product from the 3' end of the promoter-primer, which extension product is complementary to the nucleic acid sequence to be detected;
(d) contacting the product of step (c) with an agent possessing 3'-5' exonuclease activity; and
(e) synthesizing an extension product from the 3' end of the sequence to be detected, which extension product is complementary to the promoter of the promoter-primer, wherein the promoter-primer of step (a) is the only primer utilized.

2. The method of claim 1 which includes digesting the nucleic acid comprising the sequence to be detected with a restriction enzyme.

3. The method of claim 1 or claim 2 wherein the synthesis of the extension products of steps (c) and (e) is accomplished using an enzyme selected from E. coli DNA polymerase I, Klenow fragment of E. coli polymerase I and T4 DNA polymerase.

4. The method of any one of the preceding claims wherein the agent of step (d) is an enzyme selected from E. coli DNA polymerase I, Klenow fragment of E. coli DNA polymerase I and T4 DNA polymerase.

5. The method of any one of the preceding claims wherein the synthesis of the extension products of step (c) and (e) is accomplished using the same agent as that utilized in step (d).

6. The method of any one of the preceding claims wherein steps (c), (d) and (e) are carried out simultaneously in the same reaction vessel.

7. A method useful for the detection of a specific nucleic acid sequence in a test sample containing nucleic acid, comprising the synthesis of a plurality of RNA transcripts from a double-stranded nucleic acid prepared according to the method of any of the preceding claims, wherein each RNA transcript comprises an RNA sequence corresponding to the specific nucleic acid sequence to be detected, and determining the presence of said RNA sequence.

8. The method of claim 7 wherein the plurality of RNA transcripts is synthesized using T7 RNA polymerase.

9. The method of claim 7 wherein the plurality of RNA transcripts is synthesized using SP6 RNA polymerase.

10. The method of any one of claims 7, 8 and 9 wherein the presence of said RNA sequence is determined by contacting the RNA transcripts under hybridizing conditions with an oligonucleotide probe selected to hybridize with a predetermined sequence within the RNA transcripts.

11. The method of claim 10 wherein the RNA transcripts are immobilized on a solid support.

## Patentansprüche

1. Verfahren zur Herstellung einer doppelstrangigen Nukleinsäure, die einen Promoter enthält, der operabel mit einer nachzuweisenden Sequenz verbunden ist, welches Verfahren umfaßt:
(a) das Vorsehen eines Oligonukleotid-Promoter-Primers;
(b) das In-Berührung-bringen des genannten Promoter-Primers mit einer Nukleinsäure, welche die nachzuweisende Sequenz aufweist, unter Bedingungen, welche die Hybridisierung des Promoter-Primers an die nachzuweisende Nukleinsäuresequenz ermöglichen;
(c) das Synthetisieren eines Extensionsprodukts vom 3'-Ende des Promoter-Primers, welches Extensionsprodukt zur nachzuweisenden Nukleinsäuresequenz komplementär ist;
(d) das In-Berührung-bringen des Produkts von Schritt (c) mit einem Agens, das 3'-5'-Exonukleaseaktivität besitzt; und
(e) das Synthetisieren eines Extensionsprodukts vom 3'-Ende der nachzuweisenden Sequenz, welches Extensionsprodukt zum Promoter des Promoter-Primers komplementär ist, worin der Promoter-Primer von Schritt (a) der einzige verwendete Primer ist.

2. Verfahren nach Anspruch 1, welches das Digerieren der die nachzuweisende Sequenz aufweisenden Nukleinsäure mit einem Restriktionsenzym einschließt.

3. Verfahren nach Anspruch 1 oder 2, worin die Synthese der Extensionsprodukte der Schritte (c) und (e) unter Verwendung eines Enzyms erreicht wird, das aus E.coli-DNA-Polymerase I, Klenow-Fragment von E.coli-Polymerase I und T4-DNA-Polymerase ausgewählt ist.

4. Verfahren nach einem der vorhergehenden Ansprüche, worin das Agens von Schritt (d) ein Enzym ist, das aus E.coli-DNA-Polymerase I, Klenow-Fragment von E.coli-DNA-Polymerase I und T4-DNA-Polymerase ausgewählt ist.

5. Verfahren nach einem der vorhergehenden Ansprüche, worin die Synthese der Extensionsprodukte von Schritt (c) und (e) unter Verwendung des gleichen Agens erreicht wird, das in Schritt (d) verwendet wird.

6. Verfahren nach einem der vorhergehenden Ansprüche, worin die Schritte (c), (d) und (e) gleichzeitig im selben Reaktionsgefäß durchgeführt werden.

7. Verfahren, das zum Nachweis einer spezifischen Nukleinsäuresequenz in einer Testprobe nützlich ist, die Nukleinsäure enthält, welches Verfahren die Synthese einer Vielzahl von RNA-Transkripten aus einer doppelstrangigen Nukleinsäure umfaßt, die nach dem Verfahren nach einem der vorhergehenden Ansprüche hergestellt wurde, worin jedes RNA-Transkript eine RNA-Sequenz aufweist, die der spezifischen nachzuweisenden Nukleinsäuresequenz entspricht, sowie das Bestimmen der Gegenwart der genannten RNA-Sequenz.

8. Verfahren nach Anspruch 7, worin die Vielzahl von RNA-Transkripten unter Verwendung von T7-RNA-Polymerase synthetisiert wird.

9. Verfahren nach Anspruch 7, worin die Vielzahl von RNA-Transkripten unter Verwendung von SP6-RNA-Polymerase synthetisiert wird.

10. Verfahren nach einem der Ansprüche 7, 8 und 9, worin die Gegenwart der genannten RNA-Sequenz durch das In-Berührung-bringen der RNA-Transkripte unter Hybridisierungsbedingungen mit einer Oligonukleotidsonde bestimmt wird, die so ausgewählt ist, daß sie mit einer vorherbestimmten Sequenz innerhalb der RNA-Transkripte hybridisiert.

11. Verfahren nach Anspruch 10, worin die RNA-Transkripte auf einem festen Träger immobilisiert werden.

## Revendications

1. Procédé de préparation d'un acide nucléique bicaténaire qui comprend un promoteur lié de manière fonctionnelle à une séquence à détecter, procédé qui comprend :
(a) la préparation d'un promoteur-amorce oligonucléotidique ;
(b) la mise en contact dudit promoteur-amorce avec un acide nucléique comprenant la séquence à détecter dans des conditions qui permettent l'hybridation du promoteur-amorce à la séquence d'acide nucléique à détecter ;
(c) la synthèse d'un produit d'allongement à partir de l'extrémité 3' du promoteur-amorce, produit d'allongement qui est complémentaire de la séquence d'acide nucléique à détecter ;
(d) la mise en contact du produit de l'étape (c) avec un agent possédant une activité d'exonucléase 3'-5' ; et
(e) la synthèse d'un produit d'allongement à partir de l'extrémité 3' de la séquence à détecter, produit d'allongement qui est complémentaire du promoteur-amorce, dans lequel le promoteur-amorce de l'étape (a) est la seule amorce utilisée.

2. Procédé suivant la revendication 1, qui comprend la digestion de l'acide nucléique comprenant la séquence à détecter avec un enzyme de restriction.

3. Procédé suivant la revendication 1 ou la revendication 2, dans lequel la synthèse des produits d'allongement des étapes (c) et (e) est conduite au moyen d'un enzyme choisi entre l'ADN-polymérase I de E. coli, le fragment de Klenow de l'ADN-polymérase I de E. coli et l'ADN-polymérase T4.

4. Procédé suivant l'une quelconque des revendications précédentes, dans lequel l'agent de l'étape (d) est un enzyme choisi entre l'ADN-polymérase I de E. coli, le fragment de Klenow de l'ADN-polymérase I de E. coli et l'ADN-polymérase T4.

5. Procédé suivant l'une quelconque des revendications précédentes, dans lequel la synthèse des produits d'allongement des étapes (c) et (e) est conduite en utilisant le même agent que celui utilisé dans l'étape (d).

6. Procédé suivant l'une quelconque des revendications précédentes, dans lequel les étapes (c), (d) et (e) sont mises en oeuvre simultanément dans le même récipient réactionnel.

7. Procédé utile pour la détection d'une séquence d'acide nucléique spécifique dans un échantillon d'essai contenant un acide nucléique, comprenant la synthèse de plusieurs transcripts d'ARN à partir d'un acide nucléique bicaténaire préparé par le procédé suivant l'une quelconque des revendications précédentes, chaque transcript d'ARN comprenant une séquence d'ARN correspondant à la séquence d'acide nucléique spécifique à détecter, et la détermination de la présence de ladite séquence d'ARN.

8. Procédé suivant la revendication 7, dans lequel l'ensemble des transcripts d'ARN est synthétisé en utilisant l'ARN-polymérase T7.

9. Procédé suivant la revendication 7, dans lequel l'ensemble des transcripts d'ARN est synthétisé en utilisant l'ARN-polymérase SP6.

10. Procédé suivant l'une quelconque des revendications 7, 8 et 9, dans lequel la présence de la séquence d'ARN est déterminée par la mise en contact des transcripts d'ARN dans des conditions d'hybridation avec une sonde oligonucléotidique choisie pour l'hybridation avec une séquence prédéterminée à l'intérieur des transcripts d'ARN.

11. Procédé suivant la revendication 10, dans lequel les transcripts d'ARN sont immobilisés sur un support solide.
